# FASCICULE DE BREVET EUROPEEN

(11) **EP 0 692 258 B1**
(45) Date de publication et mention de la délivrance du brevet: **02.01.1997**
(21) Numéro de dépôt: 95401357.9
(22) Date de dépôt: 12.06.1995
(51) Int. Cl.: A61K 35/78, A61K 7/48

(54) **Extrait de graine de pervenche et composition le contenant**
Extrakt von Immergrünsamen und ihn enthaltende Zusammensetzung
Periwinkle seed extract and composition containing it

(30) Priorité: 11.07.1994 FR 9408566
(43) Date de publication de la demande: 17.01.1996
(73) Titulaire: L'OREAL, S.A., 75008 Paris (FR)
(72) Inventeur: Fodor, Pierre, F-92380 Garches (FR); Guth, Gérard, F-95160 Montmorency (FR); Maurin, Emmanuelle, F-78000 Versailles (FR)
(74) Mandataire: Lhoste, Catherine

(56) Documents cités:
- THE JOURNAL OF THE OIL TECHNOLOGISTS' ASSOCIATION OF INDIA, vol.19, no.3, Juillet 1987 pages 63 - 64 S.P. GARG ET AL. 'RICINOLEIC ACID IN VINCA ROSEA SEED OIL.'

## Description

L'invention a pour objet un extrait de graine de pervenche et son utilisation pour stimuler la respiration cellulaire ainsi que la croissance cellulaire, et pour protéger des radicaux libres, notamment dans les domaines cosmétique et/ou dermatologique. Plus spécialement, cet extrait est utilisé dans une composition destinée à prévenir et/ou à lutter contre le vieillissement cutané et/ou à protéger la peau, les cheveux et/ou les muqueuses.

La pervenche est une plante des lieux ombragés, à fleurs bleues à lobes étalés et aux feuilles luisantes. On distingue deux grandes familles de pervenches, la pervenche d'Amérique ou *Vinca rosea*, et la pervenche tropicale ou *Catharanthus* *roseus*. Il est connu que les feuilles et les tiges de pervenche contiennent des alcaloïdes qui ont des propriétés pharmacologiques intéressantes. De ce fait, les pervenches sont utilisées par exemple dans des préparations antidiarrhéiques, antihémorragiques, et pour empêcher les montées de lait. On utilise notamment les alcaloïdes isolées de la pervenche d'Amérique pour le traitement de la maladie d'Hodgkin ou de certaines tumeurs malignes.

Les graines de pervenche n'ont jamais fait l'objet, jusqu'à ce jour, de recherches en vue de déterminer des propriétés particulières.

La demanderesse a maintenant trouvé de manière inattendue que l'on peut obtenir, à partir des graines de pervenche, un extrait qui présente des propriétés tout à fait intéressantes, notamment dans les domaines cosmétique et dermatologique. On entend par extrait une substance extraite par une opération physique, chimique et/ou biotechnologique.

En outre, la demanderessse a trouvé que, contrairement aux tiges et aux feuilles, cet extrait ne contenait aucun alcaloïde.

Aussi, l'invention a pour objet un extrait isolé de la graine de pervenche renfermant des protéines, des acides aminés, des acides organiques et des éléments nutritifs, cet extrait étant notamment obtenu par bioconversion.

Cet extrait contient en particulier, des protéines, des acides aminés (acide glutamique, proline, lysine, valine, leucine, acide aspartique et surtout méthionine), des acides organiques (acide lactique) et des sucres (lactose).

Selon une forme préférée de réalisation de l'invention, on utilise, pour obtenir l'extrait, des graines de pervenche tropicale, et, plus spécialement, la graine de pervenche de Madagascar.

Selon l'invention, l'extrait est obtenu par bioconversion des graines de pervenche broyées.

L'invention a également pour objet un procédé pour obtenir un extrait de graine de pervenche, caractérisé en ce qu'il consiste à broyer les graines de pervenche, à les soumettre à une prédigestion enzymatique en présence d'amylase et/ou de cellulase, puis à une fermentation en présence de micro-organismes, et à filtrer.

Selon une réalisation préférée de l'invention, les micro-organismes utilisés pour la fermentation sont des micro-organismes lactiques appelés Lactobacilles.

L'invention a encore pour objet une composition comprenant un extrait aqueux de graine de pervenche.

L'invention a aussi pour objet une composition comprenant un extrait de graine de pervenche obtenu par le procédé défini ci-dessus.

Selon une forme de réalisation préférée, la composition selon l'invention comprend un milieu cosmétiquement et/ou dermatologiquement acceptable.

La demanderesse a trouvé de manière inattendue que l'extrait selon l'invention présentait la propriété de stimuler la respiration cellulaire mitochondriale et la croissance cellulaire des fibroblastes et des kératinocytes. De plus, cet extrait a une activité de protection contre les radicaux libres, notamment oxygénès. Par radicaux libres oxygénés, on entend toutes espèces réactives de l'oxygène non moléculaire, et notamment l'oxygène singulet.

Aussi, l'invention se rapporte encore à une composition pour stimuler la respiration cellulaire et/ou la croissance cellulaire et/ou empêcher la formation de radicaux libres, caractérisée ce qu'elle consiste en une composition telle que définie ci-dessus.

L'invention se rapporte également à une composition pour prévenir et/ou lutter contre le vieillissement cutané et/ou pour protéger et/ou nourrir la peau, les cheveux et/ou les muqueuses, notamment contre les effets nocifs et/ou inesthétiques provoqués par les radicaux libres, caractérisée ce qu'elle consiste en une composition telle que définie ci-dessus.

L'invention a en outre pour objet l'utilisation de l'extrait de graine de pervenche tel que défini ci-dessus dans une composition cosmétique et/ou dermatologique destinée à stimuler la respiration cellulaire et/ou la croissance cellulaire et/ou à empêcher la formation des radicaux libres.

L'invention a aussi pour objet l'utilisation de l'extrait de graine de pervenche tel que défini ci-dessus dans une composition cosmétique et/ou dermatologique destinée à prévenir et/ou à lutter contre le vieillissement cutané et/ou à protéger et/ou nourrir la peau, les cheveux et/ou les muqueuses, notamment contre les effets nocifs et/ou inesthétiques provoqués par les radicaux libres.

La présente invention a en outre pour objet un procédé pour prévenir et/ou lutter contre le vieillissement cutané et/ou protéger et/ou nourrir la peau, les cheveux et/ou les muqueuses, caractérisé en ce que l'on applique sur la peau, les cheveux et/ou les muqueuses une composition telle que définie ci-dessus.

L'extrait de graine de pervenche selon l'invention peut par exemple être utilisé dans une composition cosmétique et/ou dermatologique en une concentration allant de 0,05 % à 15 % en poids, et de préférence de 0,1 % à 10 % en poids, et encore mieux de 0,5 à 10 % en poids par rapport au poids total de la composition.

Les compositions selon l'invention peuvent se présenter sous toutes les formes galéniques normalement utilisées pour une application topique, injectable ou ingérable, destinées particulièrement aux domaines cosmétique et/ou dermatologique. En particulier, les compositions peuvent se présenter sous forme de solutions aqueuses, alcooliques ou hydroalcooliques, sous forme de crèmes, de gels hydrophiles ou lipophiles, d'émulsions eau-dans-huile ou huile-dans-eau, de crèmes ou de gels aptes à mousser, de compositions sous forme d'aérosol, ou encore sous forme de microgranulés, de poudres ou de dispersions vésiculaires de type ionique et/ou non ionique. Ces compositions sont préparées selon les méthodes usuelles des domaines considérés.

Les quantités des différents constituants des compositions selon l'invention sont celles classiquement utilisées dans les domaines cosmétique ou pharmaceutique ou de l'hygiène.

Ces compositions constituent notamment des compositions de nettoyage, de protection, de traitement ou de soin pour le visage, pour le cou, pour les mains ou pour le corps, (par exemple crèmes de jour, crèmes de nuit, crèmes démaquillantes, crèmes ou huiles solaires, laits de nettoyage, laits de démaquillage, des laits corporels), des compositions de maquillage (par exemple fonds de teint), des compositions de bronzage artificiel, ou des compositions pour le bain.

Les compositions selon l'invention peuvent également consister en des préparations solides constituant des savons ou des pains de nettoyage.

Elles peuvent être également utilisées dans diverses compositions pour les cheveux, et notamment des shampooings, des lotions de mise en plis, des lotions traitantes, des crèmes ou des gels coiffants, des compositions de teintures, des lotions ou des gels antichute.

Les compositions cosmétiques de l'invention peuvent aussi être à usage buccodentaire et se présenter par exemple sous forme d'une pâte dentifrice.

Lorsque la composition de l'invention est une émulsion, la proportion de corps gras peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique. Les émulsionnants peuvent être présents, dans la composition, en une proportion allant de 0,3 % à 30 % en poids, et de préférence de 0,5 à 30 % en poids par rapport au poids total de la composition. L'émulsion peut, en outre, contenir des vésicules lipidiques.

De façon connue, la composition cosmétique de l'invention peut contenir également des adjuvants habituels dans le domaine cosmétique, tels que les gélifiants hydrophiles ou lipophiles, les actifs hydrophiles ou lipophiles, les conservateurs, les antioxydants, les solvants (alcools), les parfums, les charges, les filtres et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme corps gras utilisables dans l'invention, on peut citer les huiles minérales, les huiles végétales (par exemple fraction liquide du beurre de karité, huile de noyau d'abricot, huile de noyau de mangues) et leurs dérivés hydrogénés (par exemple huile de palme hydrogénée), les huiles animales, les huiles de synthèse (par exemple triglycérides caprique/caprylique), les huiles siliconées (par exemple cyclométhicones et diméthicones) et les huiles fluorées. On peut ajouter à ces huiles d'autres corps gras tels que les alcools gras (par exemple alcool cétylique), les acides gras (par exemple acide stéarique) et les cires (par exemple cire végétale).

Comme émulsionnants utilisables dans l'invention, on peut citer à titre d'exemple le cétéarylglucoside vendu sous la dénomination Montanov 68 par la société Seppic, ou le mélange de cocoate de sucrose et de stéarate de sorbitan, vendu sous la dénomination Arlatone 2121 par la société ICI.

Comme gélifiants hydrophiles, on peut citer par exemple les polymères carboxyvinyliques (carbomer), les copolymères acryliques tels que les copolymères d'acrylates/alkylacrylates, les polyacrylamides, les polysaccharides tels que l'hydroxyéthylcellulose, les gommes naturelles (par exemple gomme de xanthane, sclerotium gum) et les argiles, et, comme gélifiants lipophiles, on peut citer les argiles modifiées comme les bentones, les sels métalliques d'acides gras comme les stéarates d'aluminium et la silice hydrophobe.

Comme actifs hydrophiles, on peut utiliser par exemple les protéines ou les hydrolysats de protéine, les acides aminés, les polyols, l'urée, l'allantoïne, les sucres et les dérivés de sucre, les vitamines et les hydroxyacides.

Comme actifs lipophiles, on peut utiliser par exemple le rétinol (vitamine A) et ses dérivés, le tocophérol (vitamine E) et ses dérivés tels que l'acétate de vitamine E, les acides gras essentiels, les céramides, les huiles essentielles, l'acide salicylique et ses dérivés.

Les exemples qui suivent sont donnés à titre illustratif afin de mieux faire comprendre l'invention. Les quantités indiquées sont des pourcentages en poids.

### EXEMPLE 1 : EXEMPLE DE PREPARATION DE L'EXTRAIT DE GRAINE DE PERVENCHE

On broie des graines de pervenche *Catharanthus roseus* dans un mortier. Le broyat obtenu est soumis à une prédigestion enzymatique avec une amylase et une cellulase dans un fermentateur du type Biolafitte, puis à une fermentation en présence de micro-organismes lactiques, cette fermentation étant réalisée pendant une semaine. Le milieu nutritif pour la fermentation contient notamment des peptones additionnés ou non d'extrait de levure et est vendu par Bio-Mérieux ou Merck. Il est maintenu à la température de 37°C et à un pH régulé entre 5 et 6. Le milieu de fermentation est ensuite filtré sous atmosphère contrôlée pour en éliminer les résidus de broyat et les micro-organismes.

On obtient un extrait aqueux comprenant 78,24 % d'eau, 12,8 % de polyol (glycérine), 4,62 % de protéines et d'acides aminés, 1 % d'acide lactique, 0,08 % de lactose, auxquels s'ajoutent les ingrédients du milieu de culture.

Cet extrait a la composition centésimale en acides aminés après hydrolyse, qui est la suivante :
- acide aspartique : 5,8 %,
- thréonine : 4,2 %,
- sérine : 5,5 %,
- acide glutamique : 22,5 %,
- proline : 10 %,
- glycine : 2,3 %,
- alanine : 3,7 %,
- valine : 6,8 %,
- cystine : 0,3 %,
- méthionine : 2,5 %
- isoleucine : 5,4 %,
- leucine : 9,6 %,
- tyrosine : 2,3 %,
- phénylalanine : 4,6 %,
- lysine : 8,5 %,
- arginine : 3,3 %
- ornithine : 0,2 %.
-

L'hydrolyse a été réalisée en autocatalyseur Hitachi L8500 par HCl 9N pendant 4 heures à 120°C.

### EXEMPLE 2 : CREME DE SOIN

| *Phase huileuse* : | |
|---|---|
| Cétéarylglucoside (vendu sous la dénomination Montanov 68 par la société Seppic) | 2,0 % |
| Alcool cétylique | 1,5 % |
| Cire végétale (vendu sous la dénomination Suma Wax par la société Noda) | 2,0 % |
| Fraction liquide de beurre de karité | 3,0 % |
| Huile de noyau d'abricot | 12,0 % |
| Huile de noyau de mangues | 2,0 % |
| Diméthicone (vendue sous la dénomination Silicone L-45 par la société Union Carbide) | 1,0 % |

| *Phase aqueuse :* | |
|---|---|
| Glycérine | 3,0 % |
| Hydroxyéthylcellulose (gélifiant) | 0,2 % |
| Sclerotium Gum (vendu sous la dénomination Amigel par la société Alban Muller) (gélifiant) | 0,5 % |
| Amidon (charger) | 0,4 % |
| Extrait de graine de pervenche obtenu dans l'exemple 1 | 2,5 % |
| Conservateurs | qs |
| Parfum | 0,2 % |
| Eau | qsp 100 % |

Le mode de préparation de l'émulsion consiste à mélanger la phase huileuse dans la phase aqueuse à une température d'environ 70°C à 80°C en agitant à la turbine.

Le produit obtenu est une émulsion huile-dans-eau qui a l'aspect d'une crème et peut être utilisée comme crème de nuit à appliquer quotidiennement.

### EXEMPLE 3 : MASQUE

| *Phase huileuse :* | |
|---|---|
| Octyldodécanol (émollient) | 6,0 % |
| Huile de noyau d'abricot | 6,0 % |
| Triglycérides carique/caprylique | 5,0 % |
| Kaolin (charge) | 3,0 % |
| Alcool cétylique | 2,0 % |
| Acétate de vitamine E | 0,5 % |
| Huile de palme hydrogénée | 6,0 % |
| Fraction liquide de beurre de karité | 5,0 % |

| *Phase aqueuse :* | |
|---|---|
| Gomme de xanthane (gélifiant) | 0,4 % |
| Cocoate de sucrose / Stéarate de sorbitan (mélange vendu sous la dénomination Arlatone 2121 par la société ICI) (émulsionnant) | 5,5 % |
| Glycérine | 3,0 % |
| Extrait de graine de pervenche obtenu dans l'exemple 1 | 3,0 % |
| Parfum | 0,3 % |
| Conservateurs | qs |
| Eau | qsp 100 % |

Le mode de préparation de l'émulsion est le même que dans l'exemple 2.

Le produit obtenu est une émulsion huile-dans-eau qui a l'aspect d'une crème. Il est utilisé de la manière habituelle pour les masques, c'est-à-dire qu'on l'applique sur le visage et qu'après un certain temps de pose, soit environ 3 à 10 min., on le rince à l'eau.

## Revendications

1. Extrait isolé de la graine de pervenche renfermant des protéines, des acides aminés, des acides organiques et des sucres, cet extrait étant obtenu par bioconversion.

2. Extrait selon la revendication 1, caractérisé en ce qu'il contient en outre un polyol.

3. Extrait selon la revendication 1 ou 2, caractérisé en ce qu'il comprend 0,08 % de sucre, 4,6 % de protéines et d'acides aminés, 14,8 % de polyol, 1 % d'acide carboxylique.

4. Extrait selon l'une quelconque des revendications précédentes, caractérisé en ce qu'il contient comme acides aminés l'acide aspartique, la thréonine, la sérine, l'acide glutamique, la proline, la glycine, l'alanine, la vaine, la cystine, la méthionine, l'isoleucine, la leucine, la tyrosine, la phénylalanine, la lysine, l'arginine, l'ornithine.

5. Extrait selon selon l'une quelconque des revendications précédentes, caractérisé en ce que la composition centésimale en acides aminés après hydrolyse est la suivante :
• acide aspartique : 5,8 %,
• thréonine : 4,2 %,
• sérine : 5,5 %,
• acide glutamique : 22,5 %,
• proline : 10 %,
• glycine : 2,3%,
• alanine : 3,7%,
• valine : 6,8%,
• cystine : 0,3 %,
• méthionine : 2,5 %
• isoleucine : 5,4 %,
• leucine : 9,6 %,
• tyrosine : 2,3%,
• phénylalanine : 4,6 %,
• lysine : 8,5%,
• arginine : 3,3%
• ornithine : 0,2 %.

6. Extrait selon l'une quelconque des revendications précédentes, caractérisé en ce que l'acide organique est l'acide lactique.

7. Extrait selon l'une quelconque des revendications précédentes, caractérisé en ce que le sucre est le lactose.

8. Procédé pour obtenir un extrait de graine de pervenche, caractérisé en ce qu'il consiste à broyer les graines de pervenche, à les soumettre à une prédigestion enzymatique avec une amylase et/ou une cellulase, puis à une fermentation en présence de micro-organismes et à filtrer.

9. Procédé selon la revendication 8, caractérisé en ce que les graines sont des graines de pervenche tropicale.

10. Procédé selon la revendication 8 ou 9, caractérisé en ce que la fermentation est réalisée en présence de micro-organismes lactiques.

11. Procédé selon l'une quelconque des revendications 8 à 10, caractérisé en ce que la fermentation est réalisée pendant une semaine.

12. Composition comprenant un extrait aqueux de graine de pervenche.

13. Composition selon la revendication 12, caractérisée en ce que l'extrait de graine contient des protéines, des acides aminés, des acides organiques et des éléments nutritifs.

14. Composition selon la revendication 12 ou 13, caractérisée en ce que l'extrait est obtenu par bioconversion.

15. Composition selon l'une quelconque des revendications 12 à 14, caractérisée en ce que l'extrait contient en outre un polyol.

16. Composition selon l'une quelconque des revendications 12 à 15, caractérisée en ce que l'extrait comprend 0,08 % de sucre, 4,6 % de protéines et d'acides aminés, 14,8 % de polyol, 1 % d'acide carboxylique.

17. Composition selon l'une quelconque des revendications 12 à 16, caractérisée en ce que l'extrait contient comme acides aminés l'acide aspartique, la thréonine, la sérine, l'acide glutamique, la proline, la glycine, l'alanine, la vaine, la cystine, la méthionine, l'isoleucine, la leucine, la tyrosine, la phénylalanine, la lysine, l'arginine, l'ornithine.

18. Composition selon l'une quelconque des revendications 12 à 17, caractérisée en ce que la composition centésimale en acides aminés après hydrolyse est la suivante :
• acide aspartique : 5,8 %,
• thréonine : 4,2 %,
• sérine : 5,5 %,
• acide glutamique : 22,5 %,
• proline : 10%,
• glycine : 2,3%,
• alanine : 3,7%,
• valine : 6,8 %,
• cystine : 0,3 %,
• méthionine : 2,5 %
• isoleucine : 5,4 %,
• leucine : 9,6%,
• tyrosine : 2,3%,
• phénylalanine : 4,6 %,
• lysine : 8,5%,
• arginine : 3,3%
• ornithine : 0,2 %.

19. Composition selon l'une quelconque des revendications 16 à 18, caractérisée en ce que l'acide organique est l'acide lactique.

20. Composition selon l'une quelconque des revendications 16 à 19, caractérisée en ce que le sucre est le lactose.

21. Composition comprenant un extrait de graine de pervenche obtenu par le procédé selon l'une quelconque des revendications 8 à 11.

22. Composition selon l'une quelconque des revendications 12 à 21, caractérisée en ce qu'elle comprend un milieu cosmétiquement et/ou dermatologiquement acceptable.

23. Composition selon l'une quelconque des revendications 12 à 22, caractérisée en ce que l'extrait est présent à une concentration allant de 0,05 % à 15 % en poids par rapport au poids total de la composition.

24. Composition pour stimuler la respiration cellulaire et/ou la croissance cellulaire et/ou empêcher la formation de radicaux libres, caractérisée en ce qu'elle consiste en une composition selon l'une quelconque des revendications 12 à 23.

25. Composition pour prévenir et/ou lutter contre le vieillissement cutané et/ou pour protéger et/ou nourrir la peau, les cheveux et/ou les muqueuses, caractérisée en ce qu'elle consiste en une composition selon l'une quelconque des revendications 12 à 23.

26. Utilisation de l'extrait de graine de pervenche selon l'une quelconque des revendications 1 à 7 dans et/ou pour la fabrication d'une composition cosmétique et/ou dermatologique destinée à stimuler la respiration cellulaire et/ou la croissance cellulaire et/ou à empêcher la formation des radicaux libres.

27. Utilisation de l'extrait de graine de pervenche selon l'une quelconque des revendications 1 à 7 dans et/ou pour la fabrication d'une composition cosmétique et/ou dermatologique destinée à prévenir et/ou à lutter contre le vieillissement cutané et/ou à protéger et/ou nourrir la peau, les cheveux et/ou les muqueuses.

28. Procédé cosmétique pour prévenir et/ou lutter contre le vieillissement cutané et/ou protéger et/ou nourrir la peau, les cheveux et/ou les muqueuses, caractérisé en ce que l'on applique sur la peau, les cheveux et/ou les muqueuses une composition telle que définie dans l'une quelconque des revendications 12 à 23.

## Claims

1. Extract isolated from periwinkle seed containing proteins, amino acids, organic acids and sugars, this extract being obtained by bioconversion.

2. Extract according to Claim 1, characterized in that it contains, in addition, a polyol.

3. Extract according to Claim 1 or 2, characterized in that it comprises 0.08 % of sugar, 4.6 % of proteins and amino acids, 14.8 % of polyol, 1 % of carboxylic acid.

4. Extract according to any one of the preceding claims, characterized in that it contains, as amino acids, aspartic acid, threonine, serine, glutamic acid, proline, glycine, alanine, valine, cystine, methionine, isoleucine, leucine, tyrosine, phenylalanine, lysine, arginine, ornithine.

5. Extract according to any one of the preceding claims, characterized in that the percentage amino acid composition after hydrolysis is as follows:
• aspartic acid: 5.8 %,
• threonine: 4.2 %,
• serine: 5.5 %,
• glutamic acid: 22.5 %,
• proline: 10 %,
• glycine: 2.3 %,
• alanine: 3.7 %,
• valine: 6.8 %,
• cystine: 0.3 %,
• methionine: 2.5 %,
• isoleucine: 5.4 %,
• leucine: 9.6 %,
• tyrosine: 2.3 %,
• phenylalanine: 4.6 %,
• lysine: 8.5 %,
• arginine: 3.3 %,
• ornithine: 0.2 %.

6. Extract according to any one of the preceding claims, characterized in that the organic acid is lactic acid.

7. Extract according to any one of the preceding claims, characterized in that the sugar is lactose.

8. Process for obtaining an extract of periwinkle seed, characterized in that it consists in grinding the periwinkle seeds, in subjecting them to an enzyme predigestion with an amylase and/or a cellulase and then to a fermentation in the presence of microorganisms, and in filtering.

9. Process according to Claim 8, characterized in that the seeds are tropical periwinkle seeds.

10. Process according to Claim 8 or 9, characterized in that the fermentation is carried out in the presence of lactic microorganisms.

11. Process according to any one of Claims 8 to 10, characterized in that the fermentation is carried out for one week.

12. Composition comprising an aqueous extract of periwinkle seed.

13. Composition according to Claim 12, characterized in that the extract of seed contains proteins, amino acids, organic acids and nutrient ingredients.

14. Composition according to Claim 12 or 13, characterized in that the extract is obtained by bioconversion.

15. Composition according to any one of Claims 12 to 14, characterized in that the extract contains, in addition, a polyol.

16. Composition according to any one of Claims 12 to 15, characterized in that the extract comprises 0.08 % of sugar, 4.6 % of proteins and amino acids, 14.8 % of polyol, 1 % of carboxylic acid.

17. Composition according to any one of Claims 12 to 16, characterized in that the extract contains, as amino acids, aspartic acid, threonine, serine, glutamic acid, proline, glycine, alanine, valine, cystine, methionine, isoleucine, leucine, tyrosine, phenylalanine, lysine, arginine, ornithine.

18. Composition according to any one of Claims 12 to 17, characterized in that the percentage amino acid composition after hydrolysis is as follows:
• aspartic acid: 5.8 %,
• threonine: 4.2 %,
• serine: 5.5 %,
• glutamic acid: 22.5 %,
• proline: 10 %,
• glycine: 2.3 %,
• alanine: 3.7 %,
• valine: 6.8 %,
• cystine: 0.3 %,
• methionine: 2.5 %,
• isoleucine: 5.4 %,
• leucine: 9.6 %,
• tyrosine: 2.3 %,
• phenylalanine: 4.6 %,
• lysine: 8.5 %,
• arginine: 3.3 %,
• ornithine: 0.2 %.

19. Composition according to any one of Claims 16 to 18, characterized in that the organic acid is lactic acid.

20. Composition according to any one of Claims 16 to 19, characterized in that the sugar is lactose.

21. Composition comprising an extract of periwinkle seed obtained by the process according to any one of Claims 8 to 11.

22. Composition according to any one of Claims 12 to 21, characterized in that it comprises a cosmetically and/or dermatologically acceptable medium.

23. Composition according to any one of Claims 12 to 22, characterized in that the extract is present at a concentration ranging from 0.05 % to 15 % by weight relative to the total weight of the composition.

24. Composition for stimulating cell respiration and/or cell growth and/or preventing free-radical formation, characterized in that it consists of a composition according to any one of Claims 12 to 23.

25. Composition for preventing and/or combating skin ageing and/or for protecting and/or nourishing the skin, hair and/or mucosae, characterized in that it consists of a composition according to any one of Claims 12 to 23.

26. Use of the extract of periwinkle seed according to any one of Claims 1 to 7 in and/or for the manufacture of a cosmetic and/or dermatological composition intended for stimulating cell respiration and/or cell growth and/or for preventing free-radical formation.

27. Use of the extract of periwinkle seed according to any one of Claims 1 to 7 in and/or for the manufacture of a cosmetic and/or dermatological composition intended for preventing and/or combating skin ageing and/or for protecting and/or nourishing the skin, hair and/or mucosae.

28. Cosmetic process for preventing and/or combating skin ageing and/or protecting and/or nourishing the skin, hair and/or mucosae, characterized in that a composition as defined in any one of Claims 12 to 23 is applied to the skin, hair and/or mucosae.

## Patentansprüche

1. Extrakt, der aus Immergrünsamen gewonnen wird und Proteine, Aminosäuren, organische Säuren und Zucker enthält, wobei der Extrakt durch biologische Umwandlung hergestellt wird.

2. Extrakt nach Anspruch 1, dadurch gekennzeichnet, daß er ferner ein Polyol enthält.

3. Extrakt nach Anspruch 1 oder 2, dadurch gekennzeichnet, daß er 0,08 % Zucker, 4,6 % Proteine und Aminosäuren, 14,8 % Polyol und 1 % Carbonsäure enthält.

4. Extrakt nach einem der vorhergehenden Ansprüche, dadurch gekennzeichnet, daß er als Aminosäuren Asparaginsäure, Threonin, Serin, Glutaminsäure, Prolin, Glycin, Alanin, Valin, Cystin, Methionin, Isoleucin, Leucin, Tyrosin, Phenylalanin, Lysin, Arginin und Ornithin enthält.

5. Extrakt nach einem der vorhergehenden Ansprüche, dadurch gekennzeichnet, daß er nach der Hydrolyse folgende auf hundert bezogene Zusammensetzung an Aminosäuren enthält:
• Asparaginsäure: 5,8 %,
• Threonin: 4,2 %,
• Serin: 5,5 %,
• Glutaminsäure: 22,5 %,
• Prolin: 10 %,
• Glycin: 2,3 %,
• Alanin: 3,7 %,
• Valin: 6,8 %,
• Cystin: 0,3 %,
• Methionin: 2,5 %,
• Isoleucin: 5,4 %,
• Leucin: 9,6 %,
• Tyrosin: 2,3 %,
• Phenylalanin: 4,6 %,
• Lysin: 8,5 %,
• Arginin: 3,3 %,
• Ornithin: 0,2 %.

6. Extrakt nach einem der vorhergehenden Ansprüche, dadurch gekennzeichnet, daß die organische Säure Milchsäure ist.

7. Extrakt nach einem der vorhergehenden Ansprüche, dadurch gekennzeichnet, daß der Zucker Lactose ist.

8. Verfahren zur Herstellung eines Extrakts von Immergrünsamen, dadurch gekennzeichnet, daß es darin besteht, die Immergrünsamen zu zerkleinern, sie einem enzymatischen Voraufschluß mit einer Amylase und/oder einer Cellulase und einer anschließenden Fermentation in Gegenwart von Mikroorganismen zu unterziehen und zu filtrieren.

9. Verfahren nach Anspruch 8, dadurch gekennzeichnet, daß die Samen Samen von tropischem Immergrün sind.

10. Verfahren nach Anspruch 8 oder 9, dadurch gekennzeichnet, daß die Fermentation in Gegenwart von Milchmikroorganismen durchgeführt wird.

11. Verfahren nach einem der Ansprüche 8 bis 10, dadurch gekennzeichnet, daß die Fermentation während einer Woche durchgeführt wird.

12. Zusammensetzung, die einen wäßrigen Extrakt von Immergrünsamen enthält.

13. Zusammensetzung nach Anspruch 12, dadurch gekennzeichnet, daß der Samenextrakt Proteine, Aminosäuren, organische Säuren und Nährstoffe enthält.

14. Zusammensetzung nach Anspruch 12 oder 13, dadurch gekennzeichnet, daß der Extrakt durch biologische Umwandlung hergestellt ist.

15. Zusammensetzung nach einem der Ansprüche 12 bis 14, dadurch gekennzeichnet, daß der Extrakt ferner ein Polyol enthält.

16. Zusammensetzung nach einem der Ansprüche 12 bis 15, dadurch gekennzeichnet, daß der Extrakt 0,08 % Zucker, 4,6 % Proteine und Aminosäuren, 14,8 % Polyol und 1 % Carbonsäure enthält.

17. Zusammensetzung nach einem der Ansprüche 12 bis 16, dadurch gekennzeichnet, daß der Extrakt als Aminosäuren Asparaginsäure, Threonin, Serin, Glutaminsäure, Prolin, Glycin, Alanin, Valin, Cystin, Methionin, Isoleucin, Leucin, Tyrosin, Phenylalanin, Lysin, Arginin und Ornithin enthält.

18. Zusammensetzung nach einem der Ansprüche 12 bis 17, da durch gekennzeichnet, daß sie nach der Hydrolye folgende auf hundert bezogene Zusammensetzung an Aminosäuren enthält:
• Asparaginsäure: 5,8 %,
• Threonin: 4,2 %,
• Serin: 5,5 %,
• Glutaminsäure: 22,5 %,
• Prolin: 10 %,
• Glycin: 2,3 %,
• Alanin: 3,7 %,
• Valin: 6,8 %,
• Cystin: 0,3 %,
• Methionin: 2,5 %,
• Isoleucin: 5,4 %,
• Leucin: 9,6 %,
• Tyrosin: 2,3 %,
• Phenylalanin: 4,6 %,
• Lysin: 8,5 %,
• Arginin: 3,3 %,
• Ornithin: 0,2 %.

19. Zusammensetzung nach einem der Ansprüche 16 bis 18, dadurch gekennzeichnet, daß die organische Säure Milchsäure ist.

20. Zusammensetzung nach einem der Ansprüche 16 bis 19, dadurch gekennzeichnet, daß der Zucker Lactose ist.

21. Zusammensetzung, die einen Extrakt von Immergrünsamen enthält, der nach dem Verfahren nach einem der Ansprüche 8 bis 11 hergestellt ist.

22. Zusammensetzung nach einem der Ansprüche 12 bis 21, dadurch gekennzeichnet, daß sie ein kosmetisch und/oder dermatologisch akzeptables Medium enthält.

23. Zusammensetzung nach einem der Ansprüche 12 bis 22, dadurch gekennzeichnet, daß der Extrakt in einer Konzentration im Bereich von 0,05 bis 15 Gew.-%, bezogen auf das Gesamtgewicht der Zusammensetzung, vorliegt.

24. Zusammensetzung, um die Zellatmung und/oder das Zellwachstum zu stimulieren und/oder die Bildung von freien Radikalen zu verhindern, dadurch gekennzeichnet, daß sie aus einer Zusammensetzung nach einem der Ansprüche 12 bis 23 besteht.

25. Zusammensetzung, um Hautalterung vorzubeugen und/oder sie zu bekämpfen und/oder die Haut, die Haare und/oder die Schleimhäute zu schützen und/oder zu nähren, dadurch gekennzeichnet, daß sie aus einer Zusammensetzung nach einem der Ansprüche 12 bis 23 besteht.

26. Verwendung des Extrakts von Immergrünsamen nach einem der Ansprüche 1 bis 7 in einer kosmetischen und/oder dermatologischen Zusammensetzung, die dazu bestimmt ist, die Zellatmung und/oder das Zellwachstum zu stimulieren und/oder die Bildung von freien Radikalen zu verhindern, und/oder zu ihrer Herstellung.

27. Verwendung des Extrakts von Immergrünsamen nach einem der Ansprüche 1 bis 7 in einer kosmetischen und/oder dermatologischen Zusammensetzung, die dazu bestimmt ist, Hautalterung vorzubeugen und/oder sie zu bekämpfen und/oder die Haut, die Haare und/oder die Schleimhäute zu schützen und/oder zu nähren, und/oder zu ihrer Herstellung.

28. Kosmetisches Verfahren, um Hautalterung vorzubeugen und/oder sie zu bekämpfen und/oder die Haut, die Haare und/oder die Schleimhäute zu schützen und/oder zu nähren, dadurch gekennzeichnet, daß es darin besteht, eine Zusammensetzung nach einem der Ansprüche 12 bis 23 auf die Haut, die Haare und/oder die Schleimhäute aufzutragen.
